# EUROPEAN PATENT SPECIFICATION

(11) **EP 0 352 375 B1**
(45) Date of publication and mention of the grant of the patent: **02.11.1994**
(21) Application number: 88306860.3
(22) Date of filing: 26.07.1988
(51) Int. Cl.: A61K 7/09, A61K 7/13

(54) **Preparation for dyeing and setting hair**
Präparat zum Färben und Fixieren von Haar
Préparation pour la coloration et le fixage des cheveux

(43) Date of publication of application: 31.01.1990
(73) Proprietor: Chang, Teh-Shan, Taipei (TW)
(72) Inventor: Chang, Teh-Shan, Taipei (TW)
(74) Representative: Kador & Partner

(56) References cited:
- EP-A- 0 260 716
- FR-A- 2 608 423
- GB-A- 1 077 758
- GB-A- 2 018 303
- GB-A- 2 018 836
- US-A- 4 630 621

## Description

This invention relates to a method for permanently waving and dyeing hair.

To dye and form a permanent wave in hair takes a long period of time and is very inconvenient for both the hairdresser and his client.

According to the conventional procedure, the setting and dyeing are carried out in two stages, e.g. with a separation of a week. For example, the dyeing agent is first formed by mixing 2.4% P-phenylenediamine, 16.7% hydrogen peroxide (35% H₂O₂ in weight) and water which is then applied to the hair. The whole procedure will take about 30 - 45 minutes, but the setting operation has to be carried out in the following week.

Conversely, if the setting operation is carried out first, it is usual to use a first agent composed of 12% ammonium thioglycolate, 1.5% potassium carbonate and water and a second agent composed of 3% potassium bromate. The whole procedure will take about 30 - 50 minutes and in addition to dyeing, will take more than 2 hours.

US-A-4 630 621 discloses a method for permanently waving and dyeing hair in accordance with the precharacterising part of claim 1. The para-phenylenediamine dye is in the second agent which contains the hydrogen peroxide. According to this prior art method it is necessary to remove the first agent or reforming agent before the second agent is applied. That is, rinsing and tedious blotting of the hair is necessary while it is on the rollers.

GB-A-2018836 discloses a method for dyeing hair by applying a first agent containing a mercaptane and subsequently without intermediate rinsing an oxidizing composition to develop the dyestuff as para-phenylenediamine.

FR-A- 2 608 423 discloses a method for relaxing curls by thioglycolic acid. Then hydrogen peroxide is applied to destroy the thioglycolic acid before it impairs the hair.

It is, therefore, an object of the present invention to provide a method for dyeing hair which allows setting in a permanent wave at the same time and which is economic to use.

The preparation for dyeing and setting hair, according to this invention, is composed of the following first and second agents:
A. The first agent contains the following constituents by weight:
1. Main setting agent, composed of 3 - 10% L-cysteine monohydrochloride monohydrate, 0.1 - 6% thioglycolic acid.
2. Dyeing agent, composed of oxidation dye such as p-phenlyenediamine and o-phenlyenediamine. P-phenlyenediamine is more frequently used. The quantity used is 0.01 - 4%.
3. Modifying agent, composed of resorcinol and the quantity used is 0.01- 1%.
4. Antioxidation agent, peferably composed of ascorbic acid and/or sodium metabisulfite. Both can be used simultaneously or individually and the quantity is 0,01- 1%, perferably 0,1 to 1%
5. Heavy metal ion sealing agent such as bisodium salt, trisodium salt or tetrasodium salt of EDTA and the quantity used is 0,01 - 1%, preferably 0,1 to 1%.
6. pH value adjusting agent, composed of monoethanolamine and/or ammonia. The pH value should be between 9 - 9.5
7. Surfactant agent, preferably composed of non-ionic, anionic, cationic or dual-ion surfactant agent. The most frequently used is Tween® 20 and the quantity used is 0.1 - 1%.
8. Solvent, preferably alcohol such as ethyl alcohol or isopropyl alcohol and/or water added up to 100%.

Several preferred embodiments of the present invention are described as follows:

| Constituent | Embodiment 1 Percentage | Embodiment 2 Percentage |
|---|---|---|
| L-cysteine monohydrochloride monohydrate solution | 3 | 6 |
| thioglycolic acid | 5.5 | 2 |
| P-phenylenediamine | 1.9 | 1.9 |
| O-phenylenediamine | 0.5 | 0.4 |
| asorbic acid | 0.5 | 1.0 |
| sodium metabisulfite | 0.5 | --- |
| EDTA 4 Na salt | 1.0 | 1.0 |
| Tween 20 | 1.0 | 1.0 |
| Alcohol | 8 | 6 |
| Water | 78.1 | 80.7 |

The above-mentioned constituents are blended well and then added to monoethanolamine and ammonia solution (1:1 weight in proportion) to adjust pH value to 9.0 to 9.5.
B. The second agent is the hydrogen peroxide solution containing 3 - 8% hydrogen peroxide and may be added with phosphorous acid to adjust the pH value to 4 so as to stabilize the hydrogen peroxide, if necessary.

The setting and the dyeing agents are blended without instability and both operations can be achieved in a short period.

The average required quantity of the preparation is 60 - 100 ml. for one person.

The procedures for carrying out permanent waving and dyeing are as follows:
1. Wash thoroughly, dry and separate the hair into different areas;
2. Apply one half of 30 - 50 ml of the first agent to all areas of the hair and then wave the hair;
3. Apply the other half of the first agent to every hair roller;
4. Wait 8 - 20 minutes.
5. Spray or apply well the second agent on the hair rollers twice, controlling the curvature and the dyeing effect.
6. Wait 15 - 25 minutes and detach the hair rollers; and
7. Other required means for hair dressing.

The wave may be maintained for 80 - 100 days and the colour of the dyed hair may be modified by adjusting the proportion of the P-phenylenediamine, resorcinol and hydrogen peroxide.

## Claims

1. A method for permanently waving and dyeing hair by applying a first agent to the hair on hair rollers, waiting for 8 to 20 minutes, and applying a second agent while the hair is on the rollers, the first agent containing 0,1 to 6 % by weight of thioglycolic acid and a further mercaptan acid in a solvent, the second agent containing 3 to 8 % by weight of hydrogen peroxide, and the dye being para- or ortho-phenylenediamine or a mixture thereof,
characterized in that
the second agent is applied without removing the first agent, and the first agent containing, besides the thioglycolic acid, 3 to 10 % by weight of L-cysteine monohydrochloride monohydrate as further mercaptan acid, the para- and/or ortho-phenylenediamine in an amount of 0,01 to 4 % by weight, 0,01 to 1 % by weight resorcinol, 0,01 to 1 % by weight antioxidation agent, 0,01 to 1 % by weight heavy metal ion sealing agent, 0,1 to 1 % by weight surfactant agent, and monoethanolamine and/or ammonia to adjust the pH to 9.0 to 9.5.

2. The method according to claim 1, wherein said antioxidation agent is ascorbic acid and/or sodium metabisulfite.

3. The method according to claim 1 or 2, wherein said heavy metal ion sealing agent is bisodium salt, trisodium salt or tetrasodium salt of EDTA.

4. The method according to claim 1, 2 or 3, wherein the surfactant agent is a non-ionic, anionic, cationic or dual-ion surfactant agent.

5. The method according to any preceding claim, wherein said solvent is ethyl alcohol, isopropanol and/or water.

## Patentansprüche

1. Verfahren zum dauerhaften Wellen und Färben der Haare durch Aufbringen eines ersten Mittels auf die Haare auf den Wicklern, 8- bis 20-minütiges Warten und Aufbringen eines zweiten Mittels, während die Haare auf den Wicklern sind, wobei das erste Mittel 0,1 bis 6 Gew.-% Thioglycolsäure und eine weitere Mercaptansäure in einem Lösungsmittel enthält, das zweite Mittel 3 bis 8 Gew.-% Wasserstoffperoxid enthält und der Farbstoff p- oder o-Phenylendiamin oder eine Mischung davon ist, **dadurch gekennzeichnet,** daß das zweite Mittel ohne Entfernung des ersten Mittels aufgebracht wird und das erste Mittel außer Thioglycolsäure 3 bis 10 Gew.-% L-Cysteinmonohydrochloridmonohydrat als weitere Mercaptansäure, p- und/oder o-Phenylendiamin in einer Menge von 0,01 bis 4 Gew.-%, 0,01 bis 1 Gew.-% Resorcin, 0,01 bis 1 Gew.-% eines Antioxidans, 0,01 bis 1 Gew.-% eines Schwermetallionen verschließenden Mittels, 0,01 bis 1 Gew.-% eines Tensides und Monoethanolamin und/oder Amoniak enthält, damit der pH-Wert auf 9,0 bis 9,5 eingestellt wird.

2. Verfahren nach Anspruch 1, wobei das Antioxidans Ascorbinsäure und/oder Natriummetabisulfit ist.

3. Verfahren nach Anspruch 1 oder 2, wobei das Schwermetallionen verschließende Mittel ein Binatriumsalz, Trinatriumsalz oder Tetranatriumsalz von EDTA ist.

4. Verfahren nach Anspruch 1, 2 oder 3, wobei das Tensid ein nichtionisches, anionisches, kationisches oder zweiionisches Tensid ist.

5. Verfahren nach einem der vorstehenden Ansprüche, wobei das Lösungsmittel Ethylalcohol, Isopropanol und/oder Wasser ist.

## Revendications

1. Procédé pour onduler et colorer des cheveux d'une manière permanente par application d'un premier agent sur les cheveux sur rouleaux à mise en plis, attente pendant un temps compris entre 8 et 20 mn, et application d'un deuxième agent alors que les cheveux sont sur les rouleaux, le premier agent contenant de 0,1 à 6 % en poids d'acide thioglycolique et un acide mercaptan supplémentaire dans un solvant, le deuxième agent contenant de 3 à 8 % en poids de peroxyde d'hydrogène, et le colorant étant de la para- ou ortho-phénylènediamine ou un mélange de ces dernières, caractérisé en ce que l'on applique le deuxième agent sans élimination du premier agent, et le premier agent contenant, en plus de l'acide thioglycolique, de 3 à 10 % en poids de monochlorhydrate de L-cystéine monohydraté comme acide mercaptan supplémentaire, la para- et/ou orthophénylènediamine selon une teneur comprise entre 0,01 et 4 % en poids, de 0,01 à 1 % en poids de résorcinol, de 0,01 à 1 % en poids d'agent antioxydant, de 0,01 à 1 % en poids d'agent d'étanchéité à ion de métal lourd, de 0,1 à 1 % en poids d'agent tensioactif et de la monoéthanolamine et/ou de l'ammoniaque pour régler le pH à une valeur comprise entre 9,0 et 9,5.

2. Procédé selon la revendication 1 dans lequel ledit agent antioxydant est l'acide ascorbique et/ou du métabisulfite de sodium.

3. Procédé selon la revendication 1 ou 2, dans lequel ledit agent d'étanchéité à ion de métal lourd est un sel bisodique, un sel trisodique ou un sel tétrasodique de l'EDTA.

4. Procédé selon la revendication 1,2 ou 3, dans lequel l'agent tensioactif est un agent tensioactif nonionique, anionique, cationique ou zwitterionique.

5. Procédé selon l'une quelconque des revendications précédentes, dans lequel ledit solvant est l'alcool éthylique, l'isopropanol et/ou l'eau.
